# EUROPÄISCHE PATENTANMELDUNG

(11) **EP 3 267 425 A1**
(43) Veröffentlichungstag der Anmeldung: **10.01.2018**
(21) Anmeldenummer: 17179663.4
(22) Anmeldetag: 04.07.2017
(51) Int. Cl.: G09B 19/00, G09B 7/00, G09B 5/14

(54) **EINRICHTUNG ZUM TRAINIEREN GEISTIGER UND/ODER KÖRPERLICHER FÄHIGKEITEN UND/ODER TÄTIGKEITEN EINES BENUTZERS**

(30) Priorität: 06.07.2016 DE 102016112359
(71) Anmelder: Wacker, Jürgen, 74564 Crailsheim (DE); Wacker, Ralf, 74589 Satteldorf (DE)
(72) Erfinder: Wacker, Jürgen, 74564 Crailsheim (DE); Wacker, Ralf, 74589 Satteldorf (DE)
(74) Vertreter: Kohl, Fabian Hanno

(57) **Zusammenfassung**

Die Erfindung betrifft eine Einrichtung (1) zum Trainieren geistiger und/oder körperlicher Fähigkeiten oder Tätigkeiten wenigstens eines Benutzers (2), umfassend:
- wenigstens eine erste Einheit (3);
- wenigstens eine an einem Körperteil des Benutzers (2) anbringbare oder anzubringende zweite Einheit (8), wobei
die erste Einheit (3) und/oder die zweite Einheit (8) wenigstens einen Signalgeber (6, 11) zur Gabe wenigstens eines Signals an wenigstens einen Benutzer (2) umfasst, und
- die erste Einheit (3) und die zweite Einheit (8) eingerichtet sind, über diesen jeweils zuordenbare oder zugeordnete Kommunikationseinrichtungen (7, 11) miteinander, insbesondere drahtlos, zu kommunizieren.

## Beschreibung

Die Erfindung betrifft eine Einrichtung zum Trainieren geistiger und/oder körperlicher Fähigkeiten oder Tätigkeiten wenigstens eines Benutzers.

Einrichtungen zum Trainieren geistiger und/oder körperlicher Fähigkeiten oder Tätigkeiten eines Benutzers sind in einer Vielzahl an unterschiedlichen funktionellen bzw. konstruktiven Konzipierungen bekannt. Wenngleich entsprechende Einrichtungen bisweilen auch das Training geistiger Fähigkeiten des oder der Benutzer ermöglichen, richtet sich das mit entsprechenden Einrichtungen durchführbare Training bis dato im Wesentlichen auf die körperliche, d. h. typischerweise sportliche, Betätigung des oder der Benutzer.

Wissenschaftliche Untersuchungen zeigen indes, dass gerade die Verknüpfung geistiger und körperlicher Aktivität in einer dahingehend kombinierten Trainingseinheit für alle Altersgruppen besonders positive Auswirkungen auf den menschlichen Organismus zeigen und daher empfehlenswert sind.

Es besteht daher ein Weiterentwicklungsbedarf entsprechender Einrichtungen nach einer, insbesondere im Hinblick auf die Möglichkeit einer Verknüpfung geistiger und körperlicher Aktivitäten im Rahmen einer Trainingseinheit ("kombinierte Trainingseinheit"), verbesserten Einrichtung zum Trainieren geistiger und/oder körperlicher Fähigkeiten oder Tätigkeiten eines Benutzers.

Der Erfindung liegt die Aufgabe zugrunde, eine, insbesondere im Hinblick auf die Möglichkeit einer Verknüpfung geistiger und körperlicher Aktivitäten im Rahmen einer Trainingseinheit, verbesserten Einrichtung zum Trainieren geistiger und/oder körperlicher Fähigkeiten oder Tätigkeiten wenigstens eines Benutzers anzugeben.

Die Aufgabe wird durch eine Einrichtung gemäß Anspruch 1 gelöst. Die hierzu abhängigen Ansprüche betreffen vorteilhafte Ausführungsformen der Einrichtung. Die Aufgabe wird zudem durch ein Verfahren gemäß Anspruch 17 gelöst.

Die hierin beschriebene Einrichtung dient im Allgemeinen zum Trainieren geistiger und/oder körperlicher Fähigkeiten oder Tätigkeiten eines oder mehrerer Benutzer. Mit der Einrichtung lässt sich sonach ein Training bzw.

Trainingseinheiten zum Trainieren geistiger und/oder körperlicher Fähigkeiten oder Tätigkeiten eines oder mehrerer Benutzer realisieren.

Die Einrichtung umfasst eine Mehrzahl an Bestandteilen. Wie sich im Weiteren ergibt, können die Bestandteile über diesen jeweils zuordenbare bzw. zugeordnete Kommunikationseinrichtungen, insbesondere bidirektional, miteinander kommunizieren. Die Bestandteile der Einrichtung können sonach miteinander kommunizierende Kommunikationspartner darstellen. Unter einer Kommunikation ist das Senden und/oder Empfangen von Daten zu verstehen. Die Kommunikation ist typischerweise drahtlos; zur Realisierung einer drahtlosen Kommunikation kommen grundsätzlich sämtliche Standards zur drahtlosen Kommunikation in Betracht; lediglich beispielhaft wird auf Bluetooth, DECT sowie entsprechende Kommunikationsprotokolle drahtloser Netzwerke verwiesen.

Ein erster wesentlicher Bestandteil der Einrichtung ist (wenigstens) eine erste Einheit. Die erste Einheit kann auch als stationäre Einheit bezeichnet bzw. erachtet werden, da diese während eines mit der Einrichtung durchgeführten Trainings typischerweise, jedoch nicht zwingend, an Ort und Stelle verbleibt. Die erste Einheit ist typischerweise auf einem Untergrund aufstellbar oder an einem Drittgegenstand (beschädigungs- bzw. zerstörungsfrei) lösbar befestigbar und verfügt hierzu über geeignete Aufstell- bzw. Befestigungselemente, d. h. z. B. Gurt-, Haken-, Klebe-, Klemm-, Krall-, Saug- oder Spannelemente. Unter einem Drittgegenstand ist z. B. ein äußerer oder innerer Gebäude- bzw. Bauwerksteil, wie z. B. eine Wand, eine Decke, etc., oder eine Sportgerätekonstruktion, wie z. B. ein Gerüst, ein Korb, ein Torrahmen, ein Netz, etc., zu verstehen.

Die erste Einheit kann einen Signalgeber umfassen, welcher zur Gabe wenigstens eines Signals an einen Benutzer und/oder einen Kommunikationspartner eingerichtet ist. Der ersten Einheit ist eine Kommunikationseinrichtung zuordenbar bzw. zugeordnet. Die Zuordnung kann funktionell, d. h. die Kommunikationseinrichtung bildet keinen strukturellen Bestandteil der ersten Einheit, oder strukturell, d. h. die Kommunikationseinrichtung bildet einen strukturellen Bestandteil der ersten Einheit, realisiert sein. Die Kommunikationseinrichtung ist zur Kommunikation mit wenigstens einem Kommunikationspartner, d. h. z. B. der zweiten Einheit bzw. einer dieser zuordenbaren bzw. zugeordneten Kommunikationseinrichtung, eingerichtet.

Ein weiterer bzw. zweiter wesentlicher Bestandteil der Einrichtung ist (wenigstens) eine zweite Einheit. Die zweite Einheit kann auch als mobile Einheit bezeichnet bzw. erachtet werden, da diese während eines mit der Einrichtung durchgeführten Trainings typischerweise, jedoch nicht zwingend, bewegt wird. Bewegungen der zweiten Einheit resultieren aus Bewegungen eines Benutzers, welche dieser im Rahmen eines mit der Einrichtung durchgeführten Trainings durchführt. Die zweite Einheit ist daher unmittelbar oder mittelbar, d. h. unter Zwischenschaltung wenigstens eines Gegenstands, an einem Körperteil des bzw. im Allgemeinen eines Benutzers anbringbar bzw. anzubringen, d. h. insbesondere befestigbar bzw. zu befestigen. Insbesondere kommt eine zumindest abschnittsweise Anbringung entlang bestimmter physiologischer Strukturen, d. h. z. B. entlang von Nervenbahnen, Meridianen, etc. in Betracht. Selbstverständlich ist es möglich, z. B. im Hinblick auf die abgebbare Signalstärke, unterschiedliche zweite Einheiten an einem Benutzer anzubringen. Die zweite Einheit kann z. B. in einen Gegenstand, insbesondere ein Kleidungsstück zur Bekleidung des Ober- oder Unterkörpers, wie z. B. eine Hose, ein T-Shirt, eine Jacke, etc., oder zur Bekleidung des Kopfs, wie z. B. ein Hut, eine Mütze, etc., integriert oder an diesem angebracht bzw. befestigt sein und derart (mittelbar) an einem Körperteil eines Benutzers anbringbar bzw. befestigbar sein. Alternativ kann die zweite Einheit über wenigstens ein Anbringungselement, d. h. z. B. ein Gurt-, Klebe-, Klemm- oder Saugelement, (unmittelbar) an einem Körperteil eines Benutzers anbringbar bzw. befestigbar sein. Jedenfalls ist die zweite Einheit strukturell so beschaffen bzw. eingerichtet, (beschädigungs- bzw. zerstörungsfrei) lösbar, gleichwohl unverlierbar an einem Körper eines Benutzers anbringbar bzw. befestigbar zu sein.

Analog zu der ersten Einheit kann auch die zweite Einheit einen Signalgeber umfassen, welcher zur Gabe wenigstens eines Signals an einen Benutzer und/oder einen Kommunikationspartner, d. h. z. B. die erste Einheit bzw. eine dieser zuordenbare bzw. zugeordnete Kommunikationseinrichtung, eingerichtet ist. Der zweiten Einheit ist ebenso eine Kommunikationseinrichtung zuordenbar bzw. zugeordnet. Die Zuordnung kann wiederum funktionell, d. h. die Kommunikationseinrichtung bildet keinen strukturellen Bestandteil der zweiten Einheit, oder strukturell, d. h. die Kommunikationseinrichtung bildet einen strukturellen Bestandteil der zweiten Einheit, realisiert sein. Die Kommunikationseinrichtung ist zur Kommunikation mit wenigstens einem Kommunikationspartner, d. h. z. B. der ersten Einheit bzw. einer dieser zuordenbaren bzw. zugeordneten Kommunikationseinrichtung, eingerichtet.

Die Signalgeber der ersten bzw. zweiten Einheit sind prinzipiell zur Gabe von von einem Benutzer mit wenigstens einem Sinnesorgan wahrnehmbaren Signalen eingerichtet. Die Signale können insofern auch als Reize bezeichnet bzw. erachtet werden. Bei den Signalen kann es sich um akustische und/oder elektrische und/oder mechanische und/oder optische und/oder geschmackliche und/oder olfaktorische und/oder thermische Signale handeln. Die Signalgeber können entsprechend zur Erzeugung akustischer und/oder elektrischer und/oder mechanischer und/oder optischer und/oder geschmacklicher, d. h. wenigstens einen Geschmacksstoff beinhaltender, und/oder olfaktorischer, d. h. wenigstens einen Duftstoff beinhaltender, und/oder taktiler und/oder thermischer Signale eingerichtete Signalerzeugungselemente, d. h. z. B. Tonausgabeelemente (akustisch), Bestromungselemente (elektrisch), Druck-, Zug-, Torsions- oder Scherungselemente (mechanisch), Bild- bzw. Lichtausgabeelemente (optisch), (düsenartige) Geschmacksausgabeelemente bzw. -verabreichungselemente, (düsenartige) Geruchsausgabeelemente (olfaktorisch), Vibrationselemente (taktil bzw. haptisch) oder Heiz- bzw. Kühlelemente (thermisch), umfassen. Die Gabe jeweiliger Signale kann (über einen bestimmten Zeitraum) kontinuierlich oder diskontinuierlich erfolgen. Die Signale können dabei statisch (unveränderlich) oder dynamisch (veränderlich) ausgegeben werden.

Die Gabe von geschmacklichen und/oder olfaktorischen Signalen kann insbesondere zu rehabilitationszwecken bei bestimmten geistigen und/oder körperlichen Erkrankungen, wie z. B. Parkinson, erreicht sein, da hierbei auch bei entsprechenden Erkrankungen vergleichsweise einfach erkennbare Reize realisiert werden.

Die erste und die zweite Einheit umfassen typischerweise jeweils ein Gehäuseteil, an oder in welchem funktionelle Komponenten, d. h. z. B. ein Signalgeber, eine Kommunikationseinrichtung, eine, typischerweise wiederaufladbare, elektrische Energiespeichereinrichtung (Batterie), sowie optionale, weiter unten näher spezifizierte Komponenten, der jeweiligen Einheit angeordnet sind. Das Gehäuseteil ist strukturell so beschaffen, dass die in diesem aufgenommenen funktionellen Komponenten der jeweiligen Einheit gegenüber äußeren, d. h. z. B. klimatischen und/oder mechanischen, Einflüssen geschützt sind. Das Gehäuseteil ist entsprechend zweckmäßig aus einem korrosions- und mechanisch stabilen Material, d. h. z. B. einem, gegebenenfalls mit Verstärkungsfasern verstärkten, Kunststoffmaterial, ausgebildet. Das Gehäuseteil kann komplett wasserdicht ausgebildet sein, um einen Einsatz unter Wasser - angesprochen ist sowohl Süß- als auch Salzwasser - zu ermöglichen. Die erste bzw. zweite Einheit ist sonach problemlos für ein Training unter verschiedensten klimatischen Bedingungen im Außen- und Innenbereich sowie im bzw. unter Wasser verwendbar.

Wie sich im Weiteren ergibt, ermöglicht die vorstehend beschriebene Grundkonfiguration der Einrichtung bzw. der dieser zugehörigen ersten und zweiten Einheiten ein kombiniertes Training geistiger und körperlicher Fähigkeiten bzw. Tätigkeiten eines Benutzers. Dies resultiert insbesondere aus der Gabe spezifischer Signale bzw. Signalfolgen über jeweilige Signalgeber der Einheiten bzw. damit verbundener geistiger und/oder körperlicher Aufgabestellungen für den Benutzer und die Kommunikation jeweiliger Bestandteile der Einrichtung, d. h. insbesondere der ersten und zweiten Einheiten, mit- bzw. untereinander.

In diesem Zusammenhang ist ergänzend zu erwähnen, dass sich die Einrichtung grundsätzlich für den Einsatz in jedweden Trainings- bzw. Übungsbereichen und für jedwede Altersgruppe eignet. Im Besonderen ist der Gesundheits- bzw. Rehabereich, insbesondere der Rehabereich degenerativer Erkrankungen wie Demenz, Parkinson, etc., der Kinder- und Jugendsportbereich, der Breiten- und Amateursportbereich sowie der Leistungs- und Spitzensportbereich angesprochen.

Die Einrichtung kann eine Aufgabenstellungserzeugungseinrichtung umfassen. Die Aufgabenstellungerzeugungseinrichtung ist zur Erzeugung einer eine von einem Benutzer im Rahmen eines Trainings geistig und/oder körperlich zu absolvierende Aufgabenstellung beschreibenden Aufgabenstellungsinformation eingerichtet. Durch eine entsprechende Aufgabenstellung sind bestimmte geistige und/oder körperliche Fähigkeiten bzw. Tätigkeiten eines Benutzers im Rahmen des Trainings und somit die Ausrichtung bzw. Gewichtung eines (kombinierten) Trainings im Hinblick auf das besondere Training geistiger und/oder körperlicher Fähigkeiten bzw. Tätigkeiten festlegbar; eine Aufgabenstellung kann die zu trainierenden geistigen und/oder körperlichen Fähigkeiten bzw. Tätigkeiten in einer bestimmten Art und Weise miteinander verknüpfen, sodass sich kombinierte Trainingseinheiten bzw. -effekte realisieren lassen. Eine Aufgabenstellung kann unmittelbar seitens der Aufgabenstellungserzeugungseinrichtung erzeugt, aus einer eine oder mehrere vorgegebene Aufgabenstellungen enthaltenden Speichereinrichtung der Aufgabenstellungserzeugungseinrichtung geladen, von einem Kommunikationspartner an die Aufgabenstellungserzeugungseinrichtung, welcher in diesem Fall eine Kommunikationseinrichtung zuordenbar bzw. zugeordnet sein kann, übertragen oder individuell von einem (weiteren) Benutzer, z. B. einem Trainer, vorgebbar bzw. vorgegeben sein.

Prinzipiell kann die Aufgabenstellungserzeugungseinrichtung einen Bestandteil der ersten oder zweiten Einheit bilden. Denkbar ist es jedoch auch, dass die Einrichtung eine dritte Einheit umfasst und die Aufgabenstellungserzeugungseinrichtung einen Bestandteil der dritten Einheit bildet. Die dritte Einheit, sofern vorhanden, kann auch als Bedieneinheit bezeichnet bzw. erachtet werden, da über diese, z. B. durch Auswahl oder Eingabe von Aufgabenstellungen, benutzerseitige Bedienhandlungen vorgenommen werden können. Auch der dritten Einheit ist eine Kommunikationseinrichtung zuordenbar bzw. zugeordnet. Die Zuordnung kann wiederum funktionell, d. h. die Kommunikationseinrichtung bildet keinen strukturellen Bestandteil der dritten Einheit, oder strukturell, d. h. die Kommunikationseinrichtung bildet einen strukturellen Bestandteil der dritten Einheit, realisiert sein. Die Kommunikationseinrichtung ist zur Kommunikation mit wenigstens einem Kommunikationspartner, d. h. z. B. der ersten und/oder zweiten Einheit bzw. diesen jeweils zuordenbaren bzw. zugeordneten Kommunikationseinrichtungen, eingerichtet. Über jeweilige Kommunikationseinrichtungen können Aufgabenstellungsinformationen zwischen den Einheiten übertragen, d. h. gesendet und/oder empfangen, werden. Konkret kann es sich bei der dritten Einheit um ein mobiles Endgerät, insbesondere ein Smartphone, ein Tablet(-PC), einen Laptop, handeln. Über die dritte Einheit kann eine Kommunikationsverbindung der Einrichtung, insbesondere der ersten bzw. zweiten Einheit, mit einem lokalen oder globalen Netzwerk ("Cloud") herstellbar sein. Die Aufgabenstellungserzeugungseinrichtung kann unabhängig von dessen konkreter Ausführung hard- und/oder softwaremäßig in dem mobilen Endgerät implementiert sein.

Eine Aufgabenstellung kann grundsätzlich eine oder mehrere von einem Benutzer im Rahmen eines Trainings mit der Einrichtung geistig bzw. körperlich zu absolvierende Tätigkeiten beinhalten. Eine Aufgabenstellung kann also auch mehrere, insbesondere unterschiedliche, geistig bzw. körperlich zu absolvierende Teilaufgaben beinhalten, welche von einem Benutzer in beliebiger oder bestimmter Ab- bzw. Reihenfolge zu absolvieren sind.

Um einen Benutzer über die jeweils zu absolvierende Aufgabenstellung in Kenntnis zu setzen, kann die Einrichtung eine Ausgabeeinrichtung umfassen. Die Ausgabeeinrichtung ist zur Ausgabe einer durch eine Aufgabenstellungsinformation beschriebenen Aufgabenstellung an einen Benutzer eingerichtet. Die Ausgabeeinrichtung kann insbesondere zur akustischen und/oder optischen Ausgabe der jeweiligen Aufgabenstellung eingerichtet sein und insofern z. B. eine Lautsprecher- und/oder Displayeinrichtung umfassen. Die Ausgabeeinrichtung kann eine funktionelle Komponente einer Einheit oder ein zu den Einheiten gesonderter Bestandteil der Einrichtung sein.

Im Weiteren werden beispielhaft mögliche Aufgabenstellungen dargestellt.

Eine Aufgabenstellung kann z. B. ein Annähern (kein mechanischer Kontakt erforderlich) an wenigstens einen, insbesondere annäherungsempfindlich ausgebildeten, Abschnitt, z. B. in Form eines Annäherungssensors, der ersten und/oder der zweiten Einheit mit wenigstens einem Körperteil, z. B. einer Gliedmaße, z. B. Arm, Bein, bzw. einem Teil einer solchen, z. B. Hand, Fuß, und/oder mit wenigstens einem Gegenstand, insbesondere einem Sportgerät, z. B. einem Ball, einem Schläger, etc., beinhalten. Gleichermaßen kann eine Aufgabenstellung ein Berühren (mechanischer Kontakt erforderlich) wenigstens eines, insbesondere berührungsempfindlich ausgebildeten, Abschnitts, z. B. in Form eines Berührungssensors, der ersten und/oder der zweiten Einheit mit wenigstens einem Körperteil, z. B. einer Gliedmaße, z. B. Arm, Bein, bzw. einem Teil einer solchen, z. B. Hand, Fuß, und/oder mit wenigstens einem Gegenstand, insbesondere einem Sportgerät, z. B. einem Ball, Schläger, etc., beinhalten.

Selbstverständlich ist es auch möglich, dass eine Aufgabenstellung ein Annähern an wenigstens einen, insbesondere annäherungsempfindlich ausgebildeten, Abschnitt mehrerer erster und/oder zweiter Einheiten in einer bestimmten Reihenfolge mit wenigstens einem Körperteil und/oder mit wenigstens einem Gegenstand, oder ein Berühren wenigstens eines, insbesondere berührungsempfindlich ausgebildeten, Abschnitts mehrerer erster und/oder zweiter Einheiten in einer bestimmten Reihenfolge mit wenigstens einem Körperteil und/oder mit wenigstens einem Gegenstand beinhaltet.

Eine Aufgabenstellung kann auch eine bestimmte geistige oder körperliche Tätigkeit des Benutzers, gegebenenfalls in Kombination mit einem Annähern oder Berühren wenigstens eines Abschnitts wenigstens einer ersten Einheit und/oder wenigstens einer zweiten Einheit mit wenigstens einem Körperteil und/oder mit wenigstens einem Gegenstand beinhalten. Einem Benutzer können sonach geistig und/oder körperlich zu absolvierende Aufgaben bzw. Teilaufgaben gestellt werden, vor, während oder nach deren Durchführung ein Annähern bzw. ein Berühren eines bestimmten Abschnitts wenigstens einer bestimmten Einheit durchzuführen ist, um die Aufgabenstellung erfolgreich zu absolvieren.

Einem Benutzer können also geistig zu absolvierende Tätigkeiten, d. h. z. B. mathematische, sprachliche, logische oder sonstige Denk- bzw.

Wissensübungen als Aufgabenstellung gestellt bzw. aufgegeben werden, vor, während oder nach deren Durchführung ein Annähern bzw. ein Berühren eines bestimmten Abschnitts wenigstens einer bestimmten Einheit mit einem Körperteil oder einem Gegenstand durchzuführen ist. Konkret kann einem Benutzer z. B. eine Rechen-, Grammatik- bzw. Vokabel- oder, Logik- oder Wissensübung als Aufgabenstellung aufgegeben werden, vor, während oder nach deren Durchführung ein Annähern bzw. ein Berühren eines bestimmten Abschnitts wenigstens einer bestimmten Einheit durchzuführen ist. Insbesondere kommt ein Annähern bzw. Berühren eines Abschnitts einer bestimmten Einheit in Frage, wenn die jeweilige Aufgabenstellung bzw. eine Teilaufgabe gelöst ist.

In diesem Zusammenhang ist anzumerken, dass die Einrichtung eine Eingabeeinrichtung, welche zur Eingabe zur Lösung wenigstens eines Teils einer (geistigen) Aufgabenstellung vorgenommener Benutzereingaben eingerichtet ist, umfassen kann. Über eine entsprechende Eingabeeinrichtung können sonach die im Hinblick z. B. auf gestellte Rechen-, Grammatik- bzw. Vokabel-, Logik- oder Wissensaufgabe benutzerseitig (geistig) erarbeiteten Lösungen eingegeben werden. Die Eingabeeinrichtung kann insbesondere zur akustischen und/oder optischen Eingabe der jeweiligen Lösung eingerichtet sein und insofern z. B. eine Mikrophon- und/oder Touchscreeneinrichtung umfassen. Die Eingabeeinrichtung kann eine funktionelle Komponente einer Einheit oder ein zu den Einheiten gesonderter Bestandteil der Einrichtung sein.

In analoger Weise können einem Benutzer körperlich zu absolvierende Tätigkeiten, d. h. z. B. Ausdauer-, Kraft- oder Koordinationsübungen als Aufgabenstellung gestellt bzw. aufgegeben werden, vor, während oder nach deren Durchführung ein Annähern bzw. ein Berühren eines bestimmten Abschnitts wenigstens einer bestimmten Einheit mit einem Körperteil oder einem Gegenstand durchzuführen ist. Konkret kann einem Benutzer z. B. eine bestimmte Anzahl an körperlichen Übungen, z. B. Kniebeugen, Liegestütze, Sit-Ups, Sprünge, etc., jeweils gegebenenfalls untereinander und/oder mit Gewichten oder ähnlich Wirkendem kombiniert, oder motorisch-koordinativen Übungen, z. B. Rechts-Links-Übungen, als Aufgabenstellung aufgegeben werden, vor, während oder nach deren Durchführung ein Annähern bzw. ein Berühren eines bestimmten Abschnitts wenigstens einer bestimmten Einheit durchzuführen ist. Insbesondere kommt ein Annähern bzw. Berühren eines Abschnitts einer bestimmten Einheit in Frage, wenn die jeweilige körperliche bzw. motorisch-koordinative Übung bzw. deren jeweilige Anzahl durchgeführt worden ist.

Eine Aufgabenstellung kann auch eine Bewegung wenigstens einer ersten Einheit und/oder wenigstens einer zweiten Einheit von einer Ausgangsposition oder -ausrichtung in wenigstens eine Zielposition oder -ausrichtung beinhalten. Im Rahmen der Absolvierung einer Aufgabenstellung kann sonach eine Überführung einer ersten und/oder zweiten Einheit in eine neue Position und/oder Ausrichtung gefordert sein. Diese Aufgabenstellung kann sowohl geistige als auch körperliche Tätigkeit beinhalten, als es z. B. aufgegeben sein kann, eine vergleichsweise schwere Einheit, beispielsweise kann eine erste Einheit hierfür mit gesondertem Gewicht ausgestattet sein, in eine neue Position sowie in eine neue Ausrichtung zu verbringen. Gegebenenfalls kann die Verbringung der Einheit in die neue Position ein Überwinden eines Hindernisses erfordern, welches Hindernis nur mit einer bestimmten Ausrichtung der Einheit überwunden werden kann. Zu denken ist z. B. an eine besonders, z. B. rundlich, geformte Öffnung, z. B. in einer Platte, einer Wand, etc., durch welche eine besonders, z. B. stab(zylinder)förmige, geformte Einheit in bestimmter Ausrichtung zu bewegen ist. Wiederum kann vor, während oder nach der Durchführung ein Annähern bzw. ein Berühren eines bestimmten Abschnitts wenigstens einer bestimmten Einheit durchzuführen sein. Insbesondere kommt ein Annähern bzw. Berühren eines Abschnitts einer bestimmten Einheit in Frage, wenn die jeweilige Verbringung der Einheit in die neue Position bzw. die neue Ausrichtung durchgeführt worden ist.

Aus vorstehenden Ausführungen ergibt sich, dass die Einrichtung eine Annäherungs- oder Berührungserfassungseinrichtung umfassen kann. Die Annäherungs- oder Berührungserfassungseinrichtung ist zur Erfassung einer Annäherung oder Berührung wenigstens eines, insbesondere annäherungs- oder berührungsempfindlich ausgebildeten, Abschnitts der ersten und/oder zweiten Einheit durch einen Körperteil eines Benutzers und/oder durch einen Gegenstand, insbesondere ein Sportgerät, und zur Erzeugung einer eine Annäherung oder Berührung wenigstens eines, insbesondere annäherungs- oder berührungsempfindlich ausgebildeten, Abschnitts der ersten und/oder zweiten Einheit durch einen Körperteil eines Benutzers und/oder einen Gegenstand beschreibenden Annäherungs- oder Berührungsinformation eingerichtet. Hierfür ist die Annäherungs- oder Berührungserfassungseinrichtung mit geeigneten, z. B. elektronisch oder mechanisch arbeitenden, Annäherungs- bzw. Berührungserfassungselementen bzw. -sensoren ausgestattet. Die Annäherungs- oder Berührungserfassungseinrichtung kann eine funktionelle Komponente der ersten und/oder zweiten und/oder dritten Einheit sein. Seitens der Annäherungs- oder Berührungserfassungseinrichtung erzeugte Annäherungs- oder Berührungsinformationen können zwischen den einzelnen Einheiten übertragen werden.

Die Einrichtung kann ferner eine Zeiterfassungseinrichtung umfassen. Die Zeiterfassungseinrichtung ist zur Erfassung einer die Zeit zwischen der Gabe eines seitens des Signalgebers der ersten oder zweiten Einheit gegebenen Signals und einer benutzerseitigen Reaktion auf das Signal und/oder der Absolvierung einer durch eine Aufgabenstellungsinformation beschriebenen Aufgabenstellung und zur Erzeugung eine die erfasste Zeit zwischen der Gabe eines seitens des Signalgebers der ersten oder zweiten Einheit gegebenen Signals und der benutzerseitigen Reaktion auf das Signal und/oder der Absolvierung der durch die Aufgabenstellungsinformation beschriebenen Aufgabenstellung beschreibenden Zeitinformation eingerichtet. Hierfür ist die Zeiterfassungseinrichtung mit geeigneten Zeiterfassungselementen bzw. - sensoren, z. B. in Form von Zeitmesseinrichtungen, ausgestattet.

Die Zeiterfassungseinrichtung ermöglicht eine Zeiterfassung, welche zur Auswertung bzw. Überwachung des jeweiligen Trainings, z. B. im Hinblick auf die Dauer bzw. Geschwindigkeit der Absolvierung der jeweiligen Aufgabenstellungen, von Bedeutung sein kann. Konkret kann über die Zeiterfassungseinrichtung z. B. erfasst werden, nach welcher Zeit ein Benutzer nach Gabe eines Signals eine Grundstellung, in welcher er z. B. eine am Körper getragene zweite Einheit oder eine mit entsprechenden Kontaktierungselementen, d. h. z. B. Druck- oder Gewichtssensoren, ausgestattete Bodenplatte, kontaktiert, verlässt, d. h. den Kontakt mit der zweiten Einheit bzw. der Bodenplatte aufhebt. Dies kann bei dynamischen Signalen selbstverständlich mit der Erfassung eines bestimmten Signals bzw. einer bestimmten Signalstärke verknüpft sein, bei welcher der Benutzer die Grundstellung verlässt. Die Zeiterfassungseinrichtung kann eine funktionelle Komponente einer Einheit oder ein zu den Einheiten gesonderter Bestandteil der Einrichtung sein. Seitens der Zeiterfassungseinrichtung erzeugte Zeitinformationen können zwischen den einzelnen Einheiten bzw. an externe Kommunikationspartner, z. B. ein lokales oder globales Netzwerk ("Cloud"), übertragen werden.

Die Einrichtung kann ferner eine Benutzerzustandserfassungseinrichtung umfassen. Die Benutzerzustandserfassungseinrichtung ist zur Erfassung eines geistigen und/oder körperlichen Benutzerzustands und zur Erzeugung einer einen erfassten geistigen und/oder körperlichen Benutzerzustand beschreibenden Benutzerzustandsinformation eingerichtet. Hierfür ist die Benutzerzustandserfassungseinrichtung mit geeigneten Benutzerzustandserfassungselementen bzw. -sensoren ausgestattet. Ein Benutzerzustand kann z. B. durch die Erfassung physiologisch-körperlicher Parameter, wie Blutdruck, Puls, Sauerstoffsättigung, etc., oder physiologisch-geistiger Parameter, wie z. B. elektrische Ströme zur Beschreibung der Aktivität des Gehirns, beschrieben werden. Die jeweiligen Benutzerzustandserfassungselemente bzw. -sensoren sind z. B. in Form von Blutdruck-, Puls-, Sauerstoffsättigungs- bzw. EEG-Messgeräten entsprechend zur Erfassung physiologisch-körperlicher bzw. physiologisch-geistiger Parameter eingerichtet. Die Benutzerzustandserfassungseinrichtung ermöglicht eine Benutzerzustandserfassung, welche zur Auswertung bzw. Überwachung des jeweiligen Trainings, z. B. im Hinblick auf Änderungen des Benutzerzustands während der Absolvierung der jeweiligen Aufgabenstellungen, von Bedeutung sein kann. Die Benutzerzustandserfassungseinrichtung kann eine funktionelle Komponente einer Einheit oder ein zu den Einheiten gesonderter Bestandteil der Einrichtung sein. Seitens der Benutzerzustandserfassungseinrichtung erzeugte Benutzerzustandsinformationen können zwischen den einzelnen Einheiten bzw. an externe Kommunikationspartner, z. B. ein lokales oder globales Netzwerk ("Cloud"), übertragen werden.

Weiterhin kann die Einrichtung eine Bewegungserfassungseinrichtung umfassen. Die Bewegungserfassungseinrichtung ist zur Erfassung von Bewegungen eines Benutzers, insbesondere im Zusammenhang mit der Absolvierung einer benutzerseitig zu absolvierenden Aufgabenstellung, und zur Erzeugung einer erfasste Bewegungen des Benutzers, insbesondere im Zusammenhang mit der Absolvierung der benutzerseitig zu absolvierenden Aufgabenstellung, beschreibenden Bewegungsinformation eingerichtet. Hierfür ist die Bewegungserfassungseinrichtung mit geeigneten Bewegungserfassungselementen bzw. -sensoren ausgestattet. Jeweilige Bewegungserfassungselemente bzw. -sensoren können z. B. als Beschleunigungs- bzw. Lagesensoren (Gyroskope bzw. Drehratensensoren) oder als optische Sensoren, d. h. z. B. Kameras mit entsprechender Bewegungsauswertemöglichkeit, ausgebildet sein. Die Bewegungserfassungseinrichtung ermöglicht eine Bewegungserfassung, welche zur Auswertung bzw. Überwachung des jeweiligen Trainings, z. B. im Hinblick auf bestimmte, gegebenenfalls gesondert zu trainierende, Bewegungen bzw. Bewegungsabläufe während der Absolvierung der jeweiligen Aufgabenstellungen, von Bedeutung sein kann. Die Bewegungserfassungseinrichtung kann eine funktionelle Komponente einer Einheit oder ein zu den Einheiten gesonderter Bestandteil der Einrichtung sein. Ist die Bewegungserfassungseinrichtung eine funktionelle Komponente der zweiten Einheit, ist diese vorzugsweise als Beschleunigungs- bzw. Lagesensor ausgebildet bzw. umfasst wenigstens einen solchen. Selbstverständlich können auch seitens der Bewegungserfassungseinrichtung erzeugte Bewegungsinformationen zwischen den einzelnen Einheiten bzw. an externe Kommunikationspartner, z. B. ein lokales oder globales Netzwerk ("Cloud"), übertragen werden.

Zur Realisierung der erwähnten Auswertung des Trainings, kann die Einrichtung eine Auswertungseinrichtung umfassen. Die Auswertungseinrichtung ist zur Auswertung einer bestimmten Information, d. h. z. B. einer Zeitinformation und/oder einer Benutzerzustandsinformation und/oder einer Bewegungsinformation, im Hinblick auf eine bestimmte Referenz- bzw. Zielgröße und zur Ausgabe einer ein Auswerteergebnis einer entsprechend ausgewerteten Information im Hinblick auf die bestimmte Referenz- bzw. Zielgröße beschreibenden Auswertungsinformation eingerichtet. Eine Referenz- bzw. Zielgröße kann z. B. eine bestimmte Zeit, ein bestimmter Benutzerzustand, z. B. ein bestimmter Pulsbereich, oder eine bestimmte Bewegungsabfolge sein, welche im Rahmen der Absolvierung der jeweiligen Aufgabenstellung eingehalten bzw. über- oder unterschritten werden soll. Bei der Absolvierung geistiger und/oder körperlicher Aufgabenstellungen können z. B. besonders kurze Zeiten oder besonders hohe körperliche Belastungen, d. h. z. B. ein hoher Puls, gefordert sein, um zu überprüfen, wie die jeweilige Aufgabenstellung unter entsprechend hohem zeitlichen Druck und/oder körperlicher Belastung bewältigt wird. Die Auswertungseinrichtung kann eine funktionelle Komponente einer Einheit oder ein zu den Einheiten gesonderter Bestandteil der Einrichtung sein. Selbstverständlich können auch seitens der Auswertungseinrichtung erzeugte Auswertungsinformationen zwischen den einzelnen Einheiten bzw. an externe Kommunikationspartner, z. B. ein lokales oder globales Netzwerk ("Cloud"), übertragen werden.

Die seitens den der Einrichtung zugehörigen Erfassungseinrichtungen, d. h. insbesondere der Zeiterfassungs-, Benutzerzustands-, Bewegungserfassungs- und Auswertungseinrichtung, erfassten Informationen, d. h. insbesondere Zeitinformationen, Benutzerzustandsinformationen, Bewegungsinformationen und Auswertungsinformationen, können von der Aufgabenstellungserzeugungseinrichtung bei der Erzeugung neuer bzw. abgeänderter Aufgabenstellungen berücksichtigt werden. Die Aufgabenstellungserzeugungseinrichtung kann also zur Erzeugung von Aufgabenstellungen in Abhängigkeit erfasster Informationen, insbesondere Zeitinformationen, Benutzerzustandsinformationen, Bewegungsinformationen und Auswertungsinformationen, eingerichtet sein. Derart lassen sich individuell gestaltete Aufgabenstellungen bzw. Trainingseinheiten erzeugen, welche das Trainingsergebnis optimieren können.

In analoger Weise können entsprechend erfasste Informationen bei der Erzeugung und Gabe von Signalen berücksichtigt werden. Mithin kann der Signalgeber der ersten bzw. zweiten Einheit zur Erzeugung von Signalen in Abhängigkeit erfasster Informationen, insbesondere erfasster Zeitinformationen, Benutzerzustandsinformationen oder Bewegungsinformationen, eingerichtet sein. Die gegebenen Signale können also im Hinblick z. B. auf erfasste Zeiten, Benutzerzustände und Bewegungen veränderlich sein.

Im Zusammenhang mit der Signalgabe ist anzumerken, dass Signale einem Benutzer unterschiedliche Informationen liefern können. Mithin können Signale z. B. als Startsignale, welche zum Starten der Absolvierung einer Aufgabenstellung gegeben werden, als Fehlersignale, welche nach unkorrekter oder unvollständiger Absolvierung einer Aufgabenstellung gegeben werden, als Bestätigungssignale, welche nach korrekter oder vollständiger Absolvierung einer Aufgabenstellung gegeben werden, oder als Stoppsignale, welche zum Beenden der Absolvierung einer Aufgabenstellung gegeben werden, dienen. Selbstverständlich kann ein Start-, Fehler-, Bestätigungs- oder Stoppsignal durch mehrere, gegebenenfalls unterschiedliche, Signale definiert sein.

Die Erfindung betrifft neben der Einrichtung auch ein Verfahren zum Trainieren geistiger und/oder körperlicher Fähigkeiten oder Tätigkeiten wenigstens eines Benutzers. Das Verfahren zeichnet sich dadurch aus, dass zu dessen Durchführung eine wie beschriebene und durch die entsprechenden Ansprüche definierte Einrichtung verwendet wird. Sämtliche Ausführungen im Zusammenhang mit der Einrichtung gelten daher analog für das Verfahren.

Die Erfindung ist anhand von Ausführungsbeispielen in den Zeichnungsfiguren näher erläutert. Dabei zeigen:
- Fig. 1: eine Prinzipdarstellung einer Einrichtung zum Trainieren geistiger und/oder körperlicher Fähigkeiten oder Tätigkeiten eines Benutzers gemäß einem Ausführungsbeispiel;
- Fig. 2 - 4: je eine Prinzipdarstellung einer zweiten Einheit gemäß einem Ausführungsbeispiel;
- Fig. 5 - 9: je eine Prinzipdarstellung unterschiedlicher mit der in Fig. 1 gezeigten Einrichtung zum Trainieren geistiger und/oder körperlicher Fähigkeiten oder Tätigkeiten eines Benutzers durchführbarer Trainingseinheiten.

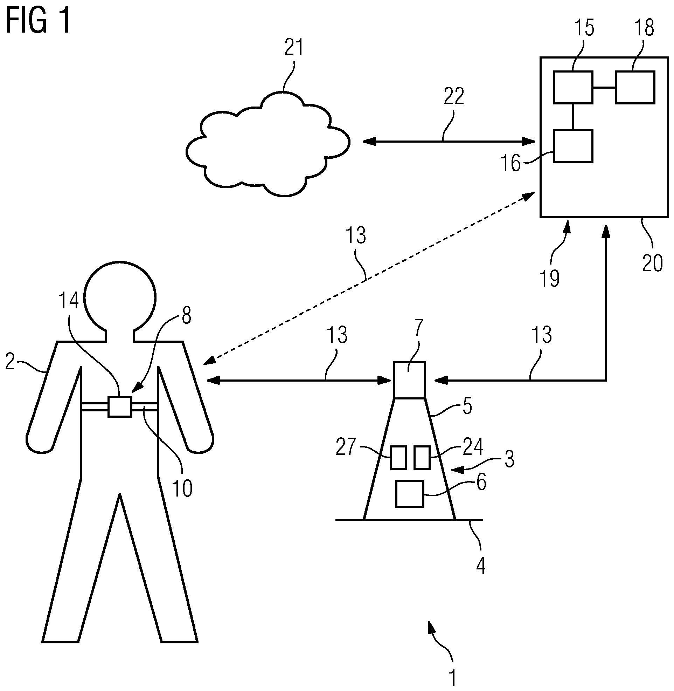

Fig. 1 zeigt eine Prinzipdarstellung einer Einrichtung 1 zum Trainieren geistiger und/oder körperlicher Fähigkeiten oder Tätigkeiten eines Benutzers 2 gemäß einem Ausführungsbeispiel.

Die Einrichtung 1 umfasst eine Mehrzahl an Bestandteilen, welche nachfolgend im Einzelnen erläutert werden:
Ein erster Bestandteil der Einrichtung 1 ist eine erste Einheit 3. Die erste Einheit 3 kann auch als stationäre Einheit bezeichnet bzw. erachtet werden, da diese während eines mit der Einrichtung 1 durchgeführten Trainings typischerweise, jedoch nicht zwingend, an Ort und Stelle verbleibt. Die im Weiteren erläuterten funktionellen Bestandteile der ersten Einheit 3 sind in einem Gehäuseteil 5 angeordnet oder ausgebildet. In dem in Fig. 1 gezeigten Ausführungsbeispiel ist die erste Einheit 3 über geeignete Aufstellelemente, hier beispielhaft in Form eines ebenen Bodens des Gehäuseteils 5, auf einem Untergrund 4 aufstellbar. Die erste Einheit 3 könnte jedoch auch an einem Gegenstand, insbesondere einer Sportgerätekonstruktion, wie z. B. einem Gerüst, einem Korb, einem Torrahmen, einem Netz, etc., (beschädigungs- bzw. zerstörungsfrei) lösbar befestigt sein. In diesem Fall verfügt die erste Einheit 3 über geeignete Befestigungselemente, d. h. z. B. Gurt-, Haken-, Klebe-, Klemm-, Krall-, Saug- oder Spannelemente.

Die erste Einheit 3 umfasst einen Signalgeber 6, welcher zur Gabe wenigstens eines Signals an einen Benutzer 2 und/oder an einen Kommunikationspartner eingerichtet ist. Der ersten Einheit 3 ist eine Kommunikationseinrichtung 7 zugeordnet. Die Zuordnung ist hier strukturell realisiert, d. h. die Kommunikationseinrichtung 7 bildet einen strukturellen Bestandteil der ersten Einheit 3 bzw. des Gehäuseteils 5. Die Kommunikationseinrichtung 7 ist zur Kommunikation mit wenigstens einem Kommunikationspartner, d. h. z. B. einer zweiten Einheit 8 bzw. einer dieser zugeordneten Kommunikationseinrichtung 9, eingerichtet. Die (bidirektionale) Kommunikation zwischen den Einheiten 3, 8 ist durch den Doppelpfeil 13 angedeutet. Ein weiterer Doppelpfeil 13 zeigt die (bidirektionale) Kommunikation zwischen der ersten Einheit 3 und einer im Weiteren erläuterten dritten Einheit 19. Durch den strichliert dargestellten Doppelpfeil ist angedeutet, dass prinzipiell auch eine (bidirektionale) Kommunikation zwischen der zweiten Einheit 8 und der dritten Einheit 19 möglich ist.

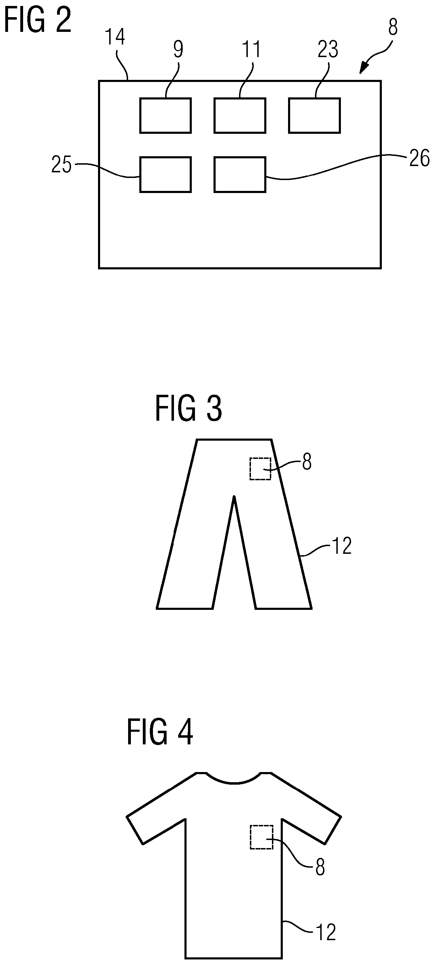

Ein weiterer bzw. zweiter Bestandteil der Einrichtung 1 ist die bereits erwähnte zweite Einheit 8 (wie strichliert angedeutet ist, umfasst die Einrichtung 1 typischerweise mehrere zweite Einheiten 8). Die in Fig. 2 in einer gesonderten Prinzipdarstellung dargestellte zweite Einheit 8 kann auch als mobile Einheit bezeichnet bzw. erachtet werden, da diese während eines mit der Einrichtung 1 durchgeführten Trainings typischerweise bewegt wird. Bewegungen der zweiten Einheit 8 resultieren aus Bewegungen des Benutzers 2, welche dieser im Rahmen eines mit der Einrichtung 1 durchgeführten Trainings durchführt. Die im Weiteren erläuterten funktionellen Bestandteile der zweiten Einheit 8 sind in einem Gehäuseteil 14 angeordnet oder ausgebildet. Die zweite Einheit 8 ist daher unmittelbar oder mittelbar, d. h. unter Zwischenschaltung wenigstens eines Gegenstands an einem Körperteil des Benutzers 2 anbringbar bzw. anzubringen, d. h. befestigbar bzw. zu befestigen. Die zweite Einheit 8 kann in einen Gegenstand, insbesondere ein Kleidungsstück 12, vgl. Fig. 3, 4, zur Bekleidung des Ober- oder Unterkörpers, wie z. B. eine Hose gemäß Fig. 3 oder ein T-Shirt gemäß Fig. 4, oder zur Bekleidung des Kopfs, wie z. B. ein Hut, eine Mütze, etc., integriert oder an diesem angebracht bzw. befestigt sein und derart (mittelbar) an einem Körperteil eines Benutzers 2 anbringbar bzw. befestigbar sein. Anhand von Fig. 1 ist ersichtlich, dass die zweite Einheit 8 über ein Anbringungselement 10, d. h. z. B. ein Gurt-, Klebe-, Klemm- oder Saugelement, (unmittelbar) an einem Körperteil eines Benutzers 2 anbringbar bzw. befestigbar ist.

Analog zu der ersten Einheit 3 umfasst auch die zweite Einheit 8 einen Signalgeber 11, welcher zur Gabe wenigstens eines Signals an einen Benutzer 2 und/oder einen Kommunikationspartner eingerichtet ist. Der zweiten Einheit 8 ist, wie erwähnt, eine Kommunikationseinrichtung 9 zugeordnet. Die Zuordnung ist auch hier strukturell realisiert, d. h. die Kommunikationseinrichtung 9 bildet einen strukturellen Bestandteil der zweiten Einheit 8 bzw. des Gehäuseteils 14. Die Kommunikationseinrichtung 9 ist zur Kommunikation mit wenigstens einem Kommunikationspartner, d. h. z. B. der ersten Einheit 3 bzw. der dieser zugeordneten Kommunikationseinrichtung 7, eingerichtet. Die Kommunikation zwischen den Einheiten 3, 8 ist, wie erwähnt, durch den Doppelpfeil 13 angedeutet.

Die Kommunikation zwischen den Einheiten 3, 8 erfolgt typischerweise drahtlos. Zur Realisierung der drahtlosen Kommunikation kommen grundsätzlich sämtliche Standards zur drahtlosen Kommunikation in Betracht; lediglich beispielhaft wird auf Bluetooth, DECT sowie entsprechende Kommunikationsprotokolle drahtloser Netzwerke verwiesen.

Die Gehäuseteile 5, 14 der jeweiligen Einheit 3, 8 sind strukturell so beschaffen, dass die in diesen aufgenommenen funktionellen Komponenten gegenüber äußeren, d. h. z. B. klimatischen und/oder mechanischen, Einflüssen geschützt sind. Die Gehäuseteile 5, 14 sind entsprechend aus einem korrosions- und mechanisch stabilen Material, d. h. z. B. einem, gegebenenfalls mit Verstärkungsfasern verstärkten, Kunststoffmaterial, ausgebildet. Die Gehäuseteile 5, 14 können komplett wasserdicht ausgebildet sein, um einen Einsatz unter Wasser - angesprochen ist sowohl Süß- als auch Salzwasser - zu ermöglichen. Die Einheiten 3, 8 sind sonach problemlos für ein Training unter verschiedensten klimatischen Bedingungen im Außen- und Innenbereich sowie im bzw. unter Wasser verwendbar.

Die Signalgeber 6, 11 der Einheiten 3, 8 sind zur Gabe von von einem Benutzer 2 mit wenigstens einem Sinnesorgan wahrnehmbaren Signalen eingerichtet. Die Signale können insofern auch als Reize bezeichnet bzw. erachtet werden. Bei den Signalen kann es sich um akustische und/oder elektrische und/oder mechanische und/oder optische und/oder geschmackliche und/oder olfaktorische und/oder taktile und/oder thermische Signale handeln. Die Signalgeber 6, 11 können entsprechend zur Erzeugung akustischer und/oder elektrischer und/oder mechanischer und/oder optischer und/oder geschmacklicher und/oder olfaktorischer und/oder taktiler und/oder thermischer Signale eingerichtete Signalerzeugungselemente, d. h. z. B. Tonausgabeelemente (akustisch), Bestromungselemente (elektrisch), Druck-, Zug-, Torsions- oder Scherungselemente (mechanisch), Bild- bzw. Lichtausgabeelemente (optisch), Geschmacksausgabeelemente (geschmacklich), Geruchsausgabeelemente (olfaktorisch), Vibrationselemente (taktil) oder Heiz- bzw. Kühlelemente (thermisch), umfassen. Die Gabe jeweiliger Signale kann (über einen bestimmten Zeitraum) kontinuierlich oder diskontinuierlich erfolgen. Die Signale können dabei statisch (unveränderlich) oder dynamisch (veränderlich) ausgegeben werden.

Die Einrichtung 1 umfasst ferner eine Aufgabenstellungserzeugungseinrichtung 15. Die Aufgabenstellungerzeugungseinrichtung 15 ist zur Erzeugung einer eine von einem Benutzer 2 im Rahmen eines Trainings geistig und/oder körperlich zu absolvierende Aufgabenstellung beschreibenden Aufgabenstellungsinformation eingerichtet. Durch eine entsprechende Aufgabenstellung sind bestimmte geistige und/oder körperliche Fähigkeiten bzw. Tätigkeiten des Benutzers 2 im Rahmen des Trainings und somit die Ausrichtung bzw. Gewichtung des Trainings festlegbar. Eine Aufgabenstellung kann unmittelbar seitens der Aufgabenstellungserzeugungseinrichtung 15 erzeugt, aus einer eine oder mehrere vorgegebene Aufgabenstellungen enthaltenden Speichereinrichtung 16 der Aufgabenstellungserzeugungseinrichtung 15 geladen, von einem Kommunikationspartner an die Aufgabenstellungserzeugungseinrichtung 16, welcher in diesem Fall eine Kommunikationseinrichtung 18 zugeordnet ist, übertragen oder individuell von einem (weiteren) Benutzer 2, z. B. einem Trainer, vorgebbar bzw. vorgegeben sein.

In dem in Fig. 1 gezeigten Ausführungsbeispiel bildet die Aufgabenstellungserzeugungseinrichtung 15 einen Bestandteil einer einen weiteren Bestandteil der Einrichtung 1 bildenden dritten Einheit 19. Bei der dritten Einheit 19 handelt es sich um ein mobiles Endgerät, d. h. z. B. um ein Smartphone, ein Tablet oder ein Laptop. Die dritte Einheit 19 kann als Bedieneinheit bezeichnet bzw. erachtet werden, da über diese, z. B. durch Auswahl oder Eingabe von Aufgabenstellungen, benutzerseitige Bedienhandlungen vorgenommen werden können. Der dritten Einheit 19 ist die Kommunikationseinrichtung 18 zugeordnet. Die Zuordnung ist wiederum strukturell, d. h. die Kommunikationseinrichtung 18 bildet einen strukturellen Bestandteil der dritten Einheit 19 bzw. eines dieser zugehörigen Gehäuseteils 20. Die Kommunikationseinrichtung 19 ist zur Kommunikation mit wenigstens einem Kommunikationspartner, d. h. z. B. der ersten und/oder zweiten Einheit 3, 8 bzw. diesen jeweils zuordenbaren bzw. zugeordneten Kommunikationseinrichtungen 7, 9, und/oder - wie durch den Doppelpfeil 22 angedeutet - einem lokalen oder globalen Netzwerk 21 ("Cloud") eingerichtet. Über jeweilige Kommunikationseinrichtungen 7, 9, 18 können Informationen, d. h. z. B. Aufgabenstellungsinformationen, zwischen den Einheiten 3, 8, 19 übertragen, d. h. gesendet und/oder empfangen, werden.

Eine durch eine Aufgabenstellungsinformation beschriebene Aufgabenstellung kann grundsätzlich eine oder mehrere von einem Benutzer 2 im Rahmen eines Trainings mit der Einrichtung 1 geistig bzw. körperlich zu absolvierende Tätigkeiten beinhalten. Eine Aufgabenstellung kann also auch mehrere, insbesondere unterschiedliche, geistig bzw. körperlich zu absolvierende Teilaufgaben beinhalten, welche von einem Benutzer 2 in beliebiger oder bestimmter Ab- bzw. Reihenfolge zu absolvieren sind.

Um einen Benutzer 2 über die jeweils zu absolvierende Aufgabenstellung in Kenntnis zu setzen, kann die Einrichtung 1 eine Ausgabeeinrichtung (nicht gezeigt) umfassen. Die Ausgabeeinrichtung ist zur Ausgabe einer durch eine Aufgabenstellungsinformation beschriebenen Aufgabenstellung an einen Benutzer 2 eingerichtet. Die Ausgabeeinrichtung kann zur akustischen und/oder optischen Ausgabe der jeweiligen Aufgabenstellung eingerichtet sein und hierfür z. B. eine Lautsprecher- und/oder Displayeinrichtung umfassen. Die Ausgabeeinrichtung kann eine funktionelle Komponente einer Einheit 3, 8 oder ein zu den Einheiten 3, 8 gesonderter Bestandteil der Einrichtung 1 sein.

Im Weiteren werden beispielhaft mögliche Aufgabenstellungen dargestellt.

Eine Aufgabenstellung kann ein Annähern an einen, insbesondere annäherungsempfindlich ausgebildeten, Abschnitt, z. B. in Form eines Annäherungs- bzw. Berührungssensors 23, z. B. der zweiten Einheit 8 mit wenigstens einem Körperteil, z. B. einer Gliedmaße, z. B. Arm, Bein, bzw. einem Teil einer solchen, z. B. Hand, Fuß, und/oder mit wenigstens einem Gegenstand, insbesondere einem Sportgerät, z. B. einem Ball, einem Schläger, etc., beinhalten. Gleichermaßen kann eine Aufgabenstellung ein Berühren eines, insbesondere berührungsempfindlich ausgebildeten, Abschnitts, z. B. in Form des Annäherungs- bzw. Berührungssensors 23, der zweiten Einheit 8 mit wenigstens einem Körperteil, z. B. einer Gliedmaße, z. B. Arm, Bein, bzw. einem Teil einer solchen, z. B. Hand, Fuß, und/oder mit wenigstens einem Gegenstand, insbesondere einem Sportgerät, z. B. einem Ball, Schläger, etc., beinhalten.

Selbstverständlich ist es auch möglich, dass eine Aufgabenstellung ein Annähern an wenigstens einen, insbesondere annäherungsempfindlich ausgebildeten, Abschnitt mehrerer erster und/oder zweiter Einheiten 3, 8 in einer bestimmten Reihenfolge mit wenigstens einem Körperteil und/oder mit wenigstens einem Gegenstand, oder ein Berühren wenigstens eines, insbesondere berührungsempfindlich ausgebildeten, Abschnitts mehrerer erster und/oder zweiter Einheiten 3, 8 in einer bestimmten Reihenfolge mit wenigstens einem Körperteil und/oder mit wenigstens einem Gegenstand beinhaltet.

Eine Aufgabenstellung kann auch eine bestimmte geistige oder körperliche Tätigkeit des Benutzers 2, gegebenenfalls in Kombination mit einem Annähern oder Berühren eines entsprechenden Abschnitts wenigstens einer Einheit 3, 8 mit wenigstens einem Körperteil und/oder mit wenigstens einem Gegenstand beinhalten. Einem Benutzer 2 können sonach geistig und/oder körperlich zu absolvierende Aufgaben bzw. Teilaufgaben gestellt werden, vor, während oder nach deren Durchführung ein Annähern bzw. ein Berühren eines bestimmten Abschnitts wenigstens einer bestimmten Einheit 3, 8 durchzuführen ist, um die Aufgabenstellung erfolgreich zu absolvieren.

Als geistige Aufgabenstellung können einem Benutzer 2 geistig zu absolvierende Tätigkeiten, d. h. z. B. mathematische, sprachliche, logische oder sonstige Denkbzw. Wissensübungen gestellt bzw. aufgegeben werden, vor, während oder nach deren Durchführung ein Annähern bzw. ein Berühren eines bestimmten Abschnitts wenigstens einer bestimmten Einheit 3, 8 mit einem Körperteil oder einem Gegenstand durchzuführen ist. Konkret kann einem Benutzer 2 z. B. eine Rechen-, Grammatik- bzw. Vokabel- oder, Logik- oder Wissensübung als Aufgabenstellung aufgegeben werden, vor, während oder nach deren Durchführung ein Annähern bzw. ein Berühren eines bestimmten Abschnitts wenigstens einer bestimmten Einheit 3, 8 durchzuführen ist. Insbesondere kommt ein Annähern bzw. Berühren eines Abschnitts einer bestimmten Einheit in Frage, wenn die jeweilige Aufgabenstellung bzw. eine Teilaufgabe gelöst ist. Dies kann, wie erwähnt, durch ein Berühren mit einer Gliedmaße oder einem Sportgerät - Beispiel ist ein gezielter Schuss oder Wurf eines Balls - erfolgen.

Die Einrichtung 1 kann eine Eingabeeinrichtung (nicht gezeigt), welche zur Eingabe zur Lösung wenigstens eines Teils einer (geistigen) Aufgabenstellung vorgenommener Benutzereingaben eingerichtet ist, umfassen. Über eine entsprechende Eingabeeinrichtung können sonach die im Hinblick z. B. auf gestellte Rechen-, Grammatik- bzw. Vokabel-, Logik- oder Wissensaufgabe benutzerseitig (geistig) erarbeiteten Lösungen eingegeben werden. Die Eingabeeinrichtung kann zur akustischen und/oder optischen Eingabe der jeweiligen Lösung eingerichtet sein und insofern z. B. eine Mikrophon- und/oder Touchscreeneinrichtung umfassen. Die Eingabeeinrichtung kann eine funktionelle Komponente einer Einheit 3, 8, oder ein zu den Einheiten 3, 8 gesonderter Bestandteil der Einrichtung 1 sein.

Als körperliche Aufgabenstellung können einem Benutzer 2 körperlich zu absolvierende Tätigkeiten, d. h. z. B. Ausdauer-, Kraft- oder Koordinationsübungen gestellt bzw. aufgegeben werden, vor, während oder nach deren Durchführung ein Annähern bzw. ein Berühren eines bestimmten Abschnitts wenigstens einer bestimmten Einheit 3, 8 mit einem Körperteil oder einem Gegenstand durchzuführen ist. Konkret kann einem Benutzer 2 z. B. eine bestimmte Anzahl an körperlichen Übungen, z. B. Kniebeugen, Liegestütze, Sit-Ups, Sprünge, etc., jeweils gegebenenfalls untereinander und/oder mit Gewichten oder ähnlich Wirkendem kombiniert, oder motorisch-koordinativen Übungen, z. B. Rechts-Links-Übungen, als Aufgabenstellung aufgegeben werden, vor, während oder nach deren Durchführung ein Annähern bzw. ein Berühren eines bestimmten Abschnitts wenigstens einer bestimmten Einheit 3, 8 durchzuführen ist. Insbesondere kommt ein Annähern bzw. Berühren eines Abschnitts einer bestimmten Einheit 3, 8 in Frage, wenn die jeweilige körperliche bzw. motorisch-koordinative Übung bzw. deren jeweilige Anzahl durchgeführt worden ist.

Eine Aufgabenstellung kann auch eine Bewegung einer Einheit 3, 8 von einer Ausgangsposition oder -ausrichtung in wenigstens eine Zielposition oder - ausrichtung beinhalten. Im Rahmen der Absolvierung einer Aufgabenstellung kann sonach eine Überführung einer Einheit 3, 8 in eine neue Position und/oder Ausrichtung gefordert sein. Diese Aufgabenstellung kann sowohl geistige als auch körperliche Tätigkeit beinhalten, als es z. B. aufgegeben sein kann, eine vergleichsweise schwere erste Einheit 3, beispielsweise kann eine erste Einheit 3 hierfür mit gesondertem Gewicht ausgestattet sein, in eine neue Position sowie in eine neue Ausrichtung zu verbringen. Gegebenenfalls kann die Verbringung der Einheit 3 in die neue Position ein Überwinden eines Hindernisses erfordern, welches Hindernis nur mit einer bestimmten Ausrichtung der Einheit 3 überwunden werden kann. Zu denken ist z. B. an eine besonders, z. B. rundlich, geformte Öffnung, z. B. in einer Platte, einer Wand, etc., durch welche eine besonders, z. B. stab(zylinder)förmige, geformte Einheit 3 in bestimmter Ausrichtung zu bewegen ist. Wiederum kann vor, während oder nach der Durchführung ein Annähern bzw. ein Berühren eines bestimmten Abschnitts wenigstens einer bestimmten Einheit 3, 8 durchzuführen sein. Insbesondere kommt ein Annähern bzw. Berühren eines Abschnitts einer bestimmten Einheit 3, 8 in Frage, wenn die jeweilige Verbringung der Einheit 3 in die neue Position bzw. die neue Ausrichtung durchgeführt worden ist.

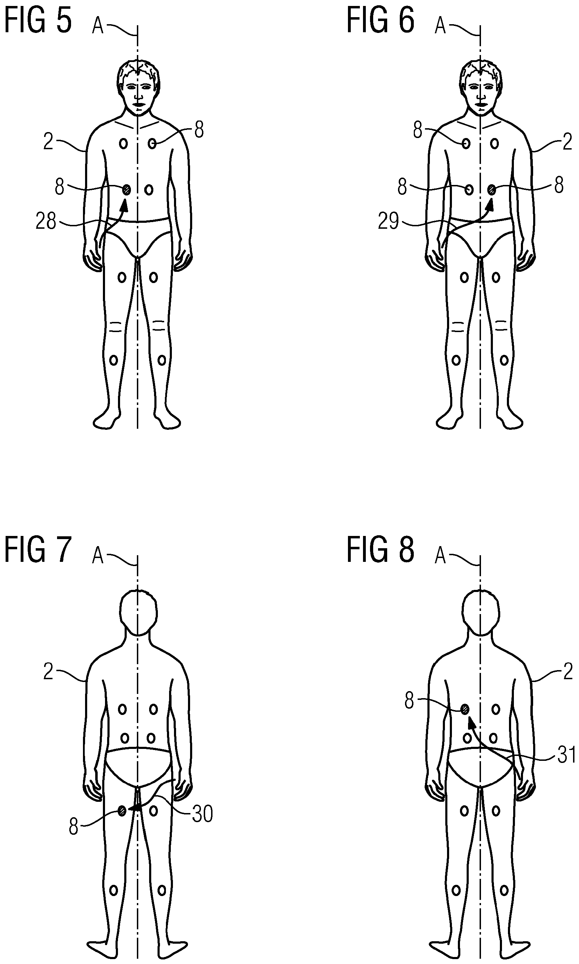

Weitere beispielhafte Aufgabenstellungen werden weiter unten im Zusammenhang mit der Erläuterung der Fig. 5 - 9 gegeben.

Zur Erfassung einer Annäherung oder Berührung eines, insbesondere annäherungs- oder berührungsempfindlich ausgebildeten, Abschnitts einer ersten und/oder zweiten Einheit 3, 8 umfasst die Einrichtung 1 eine Annäherungs- oder Berührungserfassungseinrichtung. Die Annäherungs- oder Berührungserfassungseinrichtung ist durch einen Körperteil eines Benutzers 2 und/oder durch einen Gegenstand, insbesondere ein Sportgerät, und zur Erzeugung einer eine Annäherung oder Berührung wenigstens eines, insbesondere annäherungs- oder berührungsempfindlich ausgebildeten, Abschnitts der ersten und/oder zweiten Einheit 3, 8 durch einen Körperteil eines Benutzers 2 und/oder einen Gegenstand beschreibenden Annäherungs- oder Berührungsinformation eingerichtet. Hierfür ist die Annäherungs- oder Berührungserfassungseinrichtung mit, z. B. elektronisch oder mechanisch arbeitenden, Annäherungs- bzw. Berührungserfassungssensoren 23 ausgestattet. Ein entsprechender Annäherungs- bzw. Berührungserfassungssensor 23 ist in den Fig. beispielhaft als funktionelle Komponente der zweiten Einheit 8 dargestellt (vgl. Fig. 2). Seitens der Annäherungs- oder Berührungserfassungseinrichtung erzeugte Annäherungs- oder Berührungsinformationen können zwischen den einzelnen Einheiten 3, 8, 19 übertragen werden.

Die Einrichtung 1 kann ferner eine Zeiterfassungseinrichtung 24 umfassen. Die Zeiterfassungseinrichtung 24 ist zur Erfassung einer die Zeit zwischen der Gabe eines seitens des Signalgebers 6, 11 der ersten oder zweiten Einheit 3, 8 gegebenen Signals und einer benutzerseitigen Reaktion auf das Signal und/oder der Absolvierung einer durch eine Aufgabenstellungsinformation beschriebenen Aufgabenstellung und zur Erzeugung eine die erfasste Zeit zwischen der Gabe eines gegebenen Signals und der benutzerseitigen Reaktion auf das Signal und/oder der Absolvierung der Aufgabenstellung beschreibenden Zeitinformation eingerichtet. Hierfür ist die Zeiterfassungseinrichtung 24 mit geeigneten Zeiterfassungselementen bzw. -sensoren, z. B. in Form von Zeitmesseinrichtungen, ausgestattet. Die Zeiterfassungseinrichtung 24 ermöglicht eine Zeiterfassung, welche zur Auswertung bzw. Überwachung des jeweiligen Trainings, z. B. im Hinblick auf die Dauer bzw. Geschwindigkeit der Absolvierung der jeweiligen Aufgabenstellungen, von Bedeutung sein kann. Die Zeiterfassungseinrichtung 24 ist in den Fig. beispielhaft als funktionelle Komponente der ersten Einheit 3 dargestellt. Seitens der Zeiterfassungseinrichtung 24 erzeugte Zeitinformationen können zwischen den einzelnen Einheiten 3, 8, 19 bzw. an externe Kommunikationspartner, z. B. das lokale oder globale Netzwerk 21 ("Cloud"), übertragen werden.

Die Einrichtung 1 kann ferner eine Benutzerzustandserfassungseinrichtung 25 umfassen. Die Benutzerzustandserfassungseinrichtung 25 ist zur Erfassung eines geistigen und/oder körperlichen Benutzerzustands und zur Erzeugung einer einen erfassten geistigen und/oder körperlichen Benutzerzustand beschreibenden Benutzerzustandsinformation eingerichtet. Hierfür ist die Benutzerzustandserfassungseinrichtung 25 mit geeigneten Benutzerzustandserfassungselementen bzw. -sensoren ausgestattet. Ein Benutzerzustand kann z. B. durch die Erfassung physiologisch-körperlicher Parameter, wie Blutdruck, Puls, Sauerstoffsättigung, etc., oder physiologisch-geistiger Parameter, wie z. B. elektrische Ströme zur Beschreibung der Aktivität des Gehirns, beschrieben werden. Die jeweiligen Benutzerzustandserfassungselemente bzw. -sensoren sind z. B. in Form von Blutdruck-, Puls-, Sauerstoffsättigungs- bzw. EEG-Messgeräten entsprechend zur Erfassung physiologisch-körperlicher bzw. physiologisch-geistiger Parameter eingerichtet. Die Benutzerzustandserfassungseinrichtung 25 ermöglicht eine Benutzerzustandserfassung, welche zur Auswertung bzw. Überwachung des jeweiligen Trainings, z. B. im Hinblick auf Änderungen des Benutzerzustands während der Absolvierung der jeweiligen Aufgabenstellungen, von Bedeutung sein kann. Die Benutzerzustandserfassungseinrichtung 25 ist in den Fig. beispielhaft als funktionelle Komponente der zweiten Einheit 8 dargestellt (vgl. Fig. 2). Seitens der Benutzerzustandserfassungseinrichtung 25 erzeugte Benutzerzustandsinformationen können zwischen den einzelnen Einheiten 3, 8 19 bzw. an externe Kommunikationspartner, z. B. das lokale oder globale Netzwerk 21 ("Cloud"), übertragen werden.

Weiterhin kann die Einrichtung 1 eine Bewegungserfassungseinrichtung 26 umfassen. Die Bewegungserfassungseinrichtung 26 ist zur Erfassung von Bewegungen des Benutzers 2, insbesondere im Zusammenhang mit der Absolvierung einer benutzerseitig zu absolvierenden Aufgabenstellung, und zur Erzeugung einer erfasste Bewegungen des Benutzers 2, insbesondere im Zusammenhang mit der Absolvierung der benutzerseitig zu absolvierenden Aufgabenstellung, beschreibenden Bewegungsinformation eingerichtet. Hierfür ist die Bewegungserfassungseinrichtung 26 mit geeigneten Bewegungserfassungselementen bzw. -sensoren ausgestattet. Jeweilige Bewegungserfassungselemente bzw. -sensoren können z. B. als Beschleunigungs- bzw. Lagesensoren (Gyroskope bzw. Drehratensensoren) oder als optische Sensoren, d. h. z. B. Kameras mit entsprechender Bewegungsauswertemöglichkeit, ausgebildet sein. Die Bewegungserfassungseinrichtung 26 ermöglicht eine Bewegungserfassung, welche zur Auswertung bzw. Überwachung des jeweiligen Trainings, z. B. im Hinblick auf bestimmte, gegebenenfalls gesondert zu trainierende, Bewegungen bzw. Bewegungsabläufe während der Absolvierung der jeweiligen Aufgabenstellungen, von Bedeutung sein kann. Die Bewegungserfassungseinrichtung 26 ist in den Fig. beispielhaft als funktionelle Komponente der zweiten Einheit 8 dargestellt (vgl. Fig. 2); die Bewegungserfassungseinrichtung 26 ist vorzugsweise als Beschleunigungs- bzw. Lagesensor ausgebildet bzw. umfasst wenigstens einen solchen. Selbstverständlich können auch seitens der Bewegungserfassungseinrichtung 26 erzeugte Bewegungsinformationen zwischen den einzelnen Einheiten 3, 8, 19 bzw. bzw. an externe Kommunikationspartner, z. B. das lokale oder globale Netzwerk 21 ("Cloud"), übertragen werden.

Zur Realisierung der erwähnten Auswertung des Trainings, kann die Einrichtung 1 eine Auswertungseinrichtung 27 umfassen. Die Auswertungseinrichtung 27 ist zur Auswertung einer bestimmten Information, d. h. z. B. einer Zeitinformation und/oder einer Benutzerzustandsinformation und/oder einer Bewegungsinformation, im Hinblick auf eine bestimmte Referenz- bzw. Zielgröße und zur Ausgabe einer ein Auswertungsergebnis einer entsprechend ausgewerteten Information im Hinblick auf die bestimmte Referenz- bzw. Zielgröße beschreibenden Auswertungsinformation eingerichtet. Eine Referenz- bzw. Zielgröße kann z. B. eine bestimmte Zeit, ein bestimmter Benutzerzustand, z. B. ein bestimmter Pulsbereich, oder eine bestimmte Bewegungsabfolge sein, welche im Rahmen der Absolvierung der jeweiligen Aufgabenstellung eingehalten bzw. über- oder unterschritten werden soll. Bei der Absolvierung geistiger und/oder körperlicher Aufgabenstellungen können z. B. besonders kurze Zeiten oder besonders hohe körperliche Belastungen, d. h. z. B. ein hoher Puls, gefordert sein, um zu überprüfen, wie die jeweilige Aufgabenstellung unter entsprechend hohem zeitlichen Druck und/oder körperlicher Belastung bewältigt wird. Die Auswertungseinrichtung 27 ist in den Fig. beispielhaft als funktionelle Komponente der ersten Einheit 8 dargestellt (vgl. Fig. 1). Selbstverständlich können auch seitens der Auswertungseinrichtung 27 erzeugte Auswertungsinformationen zwischen den einzelnen Einheiten bzw. bzw. an externe Kommunikationspartner, z. B. das lokale oder globale Netzwerk 21 ("Cloud"), übertragen werden.

Die seitens den der Einrichtung 1 zugehörigen Erfassungseinrichtungen, d. h. insbesondere der Zeiterfassungseinrichtung 24, der Benutzerzustandseinrichtung 25, der Bewegungserfassungseinrichtung 26 und der Auswertungseinrichtung 27, erfassten Informationen, d. h. insbesondere Zeitinformationen, Benutzerzustandsinformationen, Bewegungsinformationen und Auswertungsinformationen, können von der Aufgabenstellungserzeugungseinrichtung 15 bei der Erzeugung neuer bzw. abgeänderter Aufgabenstellungen berücksichtigt werden. Die Aufgabenstellungserzeugungseinrichtung 15 kann also zur Erzeugung von Aufgabenstellungen in Abhängigkeit erfasster Informationen, insbesondere Zeitinformationen, Benutzerzustandsinformationen, Bewegungsinformationen und Auswertungsinformationen, eingerichtet sein. Derart lassen sich individuell gestaltete Aufgabenstellungen bzw. Trainingseinheiten erzeugen, welche das Trainingsergebnis optimieren können.

In analoger Weise können entsprechend erfasste Informationen bei der Erzeugung und Gabe von Signalen berücksichtigt werden. Mithin können die Signalgeber 6, 11 der Einheiten 3, 8 zur Erzeugung von Signalen in Abhängigkeit erfasster Informationen, insbesondere erfasster Zeitinformationen, Benutzerzustandsinformationen oder Bewegungsinformationen, eingerichtet sein. Die gegebenen Signale können also im Hinblick z. B. auf erfasste Zeiten, Benutzerzustände und Bewegungen veränderlich sein.

Im Zusammenhang mit der Signalgabe ist anzumerken, dass Signale einem Benutzer unterschiedliche Informationen liefern können. Mithin können Signale z. B. als Startsignale, welche zum Starten der Absolvierung einer Aufgabenstellung gegeben werden, als Fehlersignale, welche nach unkorrekter oder unvollständiger Absolvierung einer Aufgabenstellung gegeben werden, als Bestätigungssignale, welche nach korrekter oder vollständiger Absolvierung einer Aufgabenstellung gegeben werden, oder als Stoppsignale, welche zum Beenden der Absolvierung einer Aufgabenstellung gegeben werden, dienen. Selbstverständlich kann ein Start-, Fehler-, Bestätigungs- oder Stoppsignal durch mehrere, gegebenenfalls unterschiedliche, Signale definiert sein.

Anhand der Fig. 5 - 9 werden weitere mögliche Aufgabenstellungen und Anbringungsmöglichkeiten zweiter Einheiten 8 erläutert.

Zunächst zeigen die Fig. 5 - 9 die Möglichkeit, dass mehrere zweite Einheiten 8 an einem Benutzer 2 angebracht sein können. Die zweiten Einheiten 8 sind hier beispielhaft symmetrisch bezüglich der durch die vertikale Linie angedeuteten Körpermittellinie (Saggitalachse) im Bereich des vorderen und hinteren Ober- und Unterkörpers des Benutzers 2 angebracht. Selbstverständlich sind andere Anordnungen zweiter Einheiten 8 möglich.

Fig. 5 zeigt eine Aufgabenstellung, gemäß welcher der Benutzer 2 nach Gabe eines Startsignals entsprechend der durch den Pfeil 28 beispielhaft angedeuteten Bewegungsrichtung eine bestimmte zweite Einheit 8 berühren bzw. sich dieser annähern muss. Das Startsignal kann durch ein haptisches Signal (Reiz) erfolgen und so lange anhalten, bis der Benutzer 2 die entsprechende zweite Einheit 8 berührt bzw. sich dieser annähert. Der Benutzer 2 ist gehalten, die entsprechende zweite Einheit 8 mit der rechten Gliedmaße, hier rechte Hand, möglichst schnell zu berühren. In einer Variante kann der Benutzer 2 gehalten sein, die entsprechende zweite Einheit 8 mit der linken Gliedmaße, hier linke Hand, möglichst schnell zu berühren. Aufgrund der Kreuzung der Körpermittellinie ist ein Training zur Verknüpfung der rechten und linken Gehirnhälfte möglich.

In Fig. 6 ist eine entsprechende Variante gezeigt, bei welcher grundsätzlich eine Kreuzung der Körpermittellinie vorzunehmen ist, vgl. Pfeil 29.

In den Fig. 7, 8 sind weitere Varianten gezeigt, bei welchen grundsätzlich eine Kreuzung der Körpermittellinie vorzunehmen ist, vgl. Pfeile 30, 31. Die zweiten Einheiten 8, welche zu berühren bzw. welchen sich anzunähern ist, befinden sich hier jedoch auf der Körperrückseite.

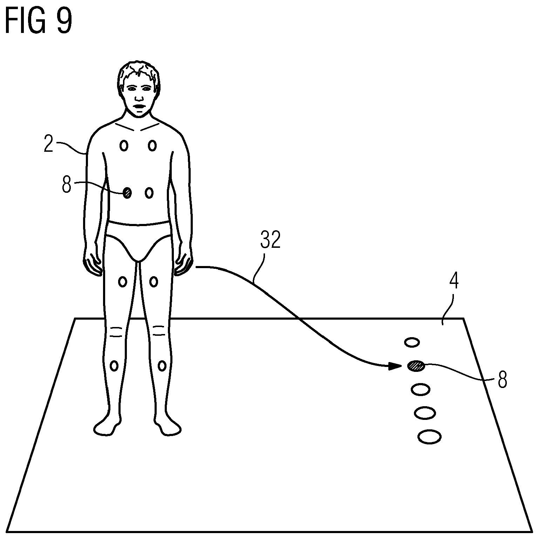

Fig. 9 zeigt eine Variante, bei welcher nach Gabe eines Signals über eine am Körper des Benutzers 2 angebrachte zweite Einheit 8 eine oder mehrere nicht am Körper des Benutzers 2 angebrachte, sondern z. B. auf einem Untergrund 4 befindliche oder an einem Gegenstand (nicht gezeigt), d. h. z. B. einem Gebäude- bzw. Bauwerksteil oder einer Sportgerätekonstruktion, befestigte zweite Einheiten 8, vgl. Pfeil 32, zu berühren sind. Selbstverständlich ist ein hierzu umgekehrtes Prinzip ebenso denkbar.

Die im Zusammenhang mit den Fig. 5 - 9 erläuterten Varianten sind untereinander kombinierbar. In allen Varianten ist auch eine Signalgabe über die erste Einheit 3 möglich.

Aus den Erläuterungen zu der Einrichtung 1 ergibt sich, dass sich mit der Einrichtung 1 ein Verfahren zum Trainieren geistiger und/oder körperlicher Fähigkeiten oder Tätigkeiten wenigstens eines Benutzers 2 implementieren lässt.

## Patentansprüche

1. Einrichtung (1) zum Trainieren geistiger und/oder körperlicher Fähigkeiten oder Tätigkeiten wenigstens eines Benutzers (2), umfassend:
- wenigstens eine erste Einheit (3);
- wenigstens eine an einem Körperteil des Benutzers (2) anbringbare oder anzubringende zweite Einheit (8), wobei
die erste Einheit (3) und/oder die zweite Einheit (8) wenigstens einen Signalgeber (6, 11) zur Gabe wenigstens eines Signals an wenigstens einen Benutzer (2) umfasst, und
- die erste Einheit (3) und die zweite Einheit (8) eingerichtet sind, über diesen jeweils zuordenbare oder zugeordnete Kommunikationseinrichtungen (7, 11) miteinander, insbesondere drahtlos, zu kommunizieren.

2. Einrichtung nach Anspruch 1, **dadurch gekennzeichnet, dass** die zweite Einheit (8) als in einen Gegenstand, insbesondere ein Kleidungsstück (12), integriert über den Gegenstand an einem Körperteil eines Benutzers (2) anbringbar oder über wenigstens ein Anbringungselement an einem Körperteil eines Benutzers (2) anbringbar ist.

3. Einrichtung nach Anspruch 1 oder 2, **dadurch gekennzeichnet, dass** die erste Einheit (3) auf einem Untergrund (4) aufstellbar oder an einem Drittgegenstand, insbesondere einer Sportgerätekonstruktion, befestigbar ist.

4. Einrichtung nach einem der vorhergehenden Ansprüche, **gekennzeichnet durch** eine Aufgabenstellungserzeugungseinrichtung (15), welche zur Erzeugung einer eine von einem Benutzer (2) geistig und/oder körperlich zu absolvierende Aufgabenstellung beschreibenden Aufgabenstellungsinformation eingerichtet ist.

5. Einrichtung nach Anspruch 4, **dadurch gekennzeichnet, dass** die Aufgabenstellungserzeugungseinrichtung (15) ein Bestandteil einer dritten Einheit (19) der Einrichtung (1) ist, wobei die dritte Einheit (19) als mobiles Endgerät, insbesondere Smartphone, Tablet-PC, Laptop, ausgebildet ist.

6. Einrichtung nach Anspruch 4 oder 5, **dadurch gekennzeichnet, dass** eine Aufgabenstellung beinhaltet:
- ein Annähern an wenigstens einen, insbesondere annäherungs- oder berührungsempfindlich ausgebildeten, Abschnitt der ersten Einheit (3) und/oder der zweiten Einheit (8) mit wenigstens einem Körperteil und/oder mit wenigstens einem Gegenstand, insbesondere einem Sportgerät, oder ein Berühren wenigstens eines, insbesondere annäherungs- oder berührungsempfindlich ausgebildeten, Abschnitts der ersten Einheit (3) und/oder der zweiten Einheit (8) mit wenigstens einem Körperteil und/oder mit wenigstens einem Gegenstand, insbesondere einem Sportgerät; und/oder
- ein Annähern an wenigstens einen, insbesondere annäherungs- oder berührungsempfindlich ausgebildeten, Abschnitt mehrerer erster und/oder zweiter Einheiten (3, 8) in einer bestimmten Reihenfolge mit wenigstens einem Körperteil und/oder mit wenigstens einem Gegenstand, oder ein Berühren wenigstens eines, insbesondere annäherungs- oder berührungsempfindlich ausgebildeten, Abschnitts mehrerer erster und/oder zweiter Einheiten (3, 8) in einer bestimmten Reihenfolge mit wenigstens einem Körperteil und/oder mit wenigstens einem Gegenstand, insbesondere einem Sportgerät; und/oder
- eine bestimmte geistige oder körperliche Tätigkeit des Benutzers (2), gegebenenfalls in Kombination mit einem Annähern oder Berühren wenigstens eines Abschnitts wenigstens einer ersten Einheit (3) und/oder wenigstens einer zweiten Einheit (8) mit wenigstens einem Körperteil und/oder mit wenigstens einem Gegenstand; und/oder
- eine Bewegung wenigstens einer ersten Einheit (3) und/oder wenigstens einer zweiten Einheit (8) von einer Ausgangsposition oder -ausrichtung in wenigstens eine Zielposition oder -ausrichtung.

7. Einrichtung nach einem der Ansprüche 4 bis 7, **gekennzeichnet durch** eine Ausgabeeinrichtung, welche zur Ausgabe einer durch eine Aufgabenstellungsinformation beschriebenen Aufgabenstellung an einen Benutzer (2) eingerichtet ist.

8. Einrichtung nach einem der Ansprüche 4 bis 7, **gekennzeichnet durch** eine Eingabeeinrichtung, welche zur Eingabe zur Lösung wenigstens eines Teils einer Aufgabenstellung vorgenommener Benutzereingaben eingerichtet ist.

9. Einrichtung nach einem der vorhergehenden Ansprüche, **gekennzeichnet durch** eine Annäherungs- oder Berührungserfassungseinrichtung, welche zur Erfassung einer Annäherung oder Berührung wenigstens eines, insbesondere annäherungs- oder berührungsempfindlich ausgebildeten, Abschnitts der ersten und/oder zweiten Einheit (3, 8) durch einen Körperteil eines Benutzers (2) und/oder einen Gegenstand, insbesondere ein Sportgerät, und zur Erzeugung einer eine Annäherung oder Berührung wenigstens eines, insbesondere annäherungs- oder berührungsempfindlich ausgebildeten, Abschnitts der ersten und/oder zweiten Einheit (3, 8) durch einen Körperteil eines Benutzers (2) und/oder einen Gegenstand beschreibenden Annäherungs- oder Berührungsinformation eingerichtet ist.

10. Einrichtung nach einem der vorhergehenden Ansprüche, **gekennzeichnet durch** eine Zeiterfassungseinrichtung (24), welche zur Erfassung einer die Zeit zwischen der Gabe eines seitens des Signalgebers (6, 11) der ersten oder zweiten Einheit (3, 8) gegebenen Signals und einer benutzerseitigen Reaktion auf das Signal und/oder der Absolvierung einer durch eine Aufgabenstellungsinformation beschriebenen Aufgabenstellung und zur Erzeugung eine die erfasste Zeit zwischen der Gabe eines seitens des Signalgebers (6, 11) der ersten oder zweiten Einheit (3, 8) gegebenen Signals und der benutzerseitigen Reaktion auf das Signal und/oder der Absolvierung der durch die Aufgabenstellungsinformation beschriebenen Aufgabenstellung beschreibenden Zeitinformation eingerichtet ist.

11. Einrichtung nach einem der vorhergehenden Ansprüche, **gekennzeichnet durch** eine Benutzerzustandserfassungseinrichtung (25), welche zur Erfassung eines geistigen und/oder körperlichen Benutzerzustands und zur Erzeugung einer einen erfassten geistigen und/oder körperlichen Benutzerzustand beschreibenden Benutzerzustandsinformation eingerichtet ist.

12. Einrichtung nach einem der vorhergehenden Ansprüche, **gekennzeichnet durch** eine Bewegungserfassungseinrichtung (26), welche zur Erfassung von Bewegungen eines Benutzers (2), insbesondere im Zusammenhang mit der Absolvierung einer benutzerseitig zu absolvierenden Aufgabenstellung, und zur Erzeugung einer erfasste Bewegungen des Benutzers (2), insbesondere im Zusammenhang mit der Lösung einer benutzerseitig zu lösenden Aufgabenstellung, beschreibenden Bewegungsinformation eingerichtet ist.

13. Einrichtung nach einem der vorhergehenden Ansprüche, **gekennzeichnet durch** eine Auswertungseinrichtung (27), welche zur Auswertung einer Zeitinformation und/oder einer Benutzerzustandsinformation und/oder einer Bewegungsinformation im Hinblick auf eine bestimmte Referenzgröße und zur Ausgabe einer ein Auswerteergebnis einer Zeiterfassungsinformation und/oder einer Benutzerzustandsinformation im Hinblick auf die bestimmte Referenzgröße beschreibenden Auswertungsinformation eingerichtet ist.

14. Einrichtung nach einem der Ansprüche 4 bis 13, **dadurch gekennzeichnet, dass** die Aufgabenstellungserzeugungseinrichtung (15) zur Erzeugung von Aufgabenstellungen in Abhängigkeit erfasster Informationen, insbesondere erfasster Zeitinformationen, Benutzerzustandsinformationen oder Bewegungsinformationen, eingerichtet ist.

15. Einrichtung nach einem der vorhergehenden Ansprüche, **dadurch gekennzeichnet, dass** der Signalgeber (6, 11) der ersten oder zweiten Einheit (3, 8) zur Erzeugung von Signalen in Abhängigkeit erfasster Informationen, insbesondere erfasster Zeitinformationen, Benutzerzustandsinformationen, oder Bewegungsinformationen, eingerichtet ist.

16. Einrichtung nach einem der vorhergehenden Ansprüche, **dadurch gekennzeichnet, dass** der Signalgeber (6, 11) der ersten oder zweiten Einheit (3, 8) zur Gabe akustischer und/oder elektrischer und/oder mechanischer und/oder optischer und/oder geschmacklicher und/oder olfaktorischer und/oder taktiler und/oder thermischer Signale eingerichtet ist.

17. Verfahren zum Trainieren geistiger und/oder körperlicher Fähigkeiten oder Tätigkeiten wenigstens eines Benutzers (2), **dadurch gekennzeichnet, dass** zur Durchführung des Verfahrens eine Einrichtung (1) nach einem der vorhergehenden Ansprüche verwendet wird.
